# EUROPEAN PATENT APPLICATION

(11) **EP 3 760 218 A1**
(43) Date of publication of application: **06.01.2021**
(21) Application number: 19382575.9
(22) Date of filing: 05.07.2019
(51) Int. Cl.: A61K 38/18, A61K 31/10, A61K 31/713, A61K 36/235, A61K 38/20, A61K 41/00, A61P 17/00, A61P 17/02

(54) **MICRO-IMMUNOTHERAPY COMPOSITIONS AND THEIR USES IN WOUND HEALING AND TISSUE REPAIR**

(71) Applicant: Labo'Life España S.A., 07330 Consell (ES); Amarilys Limited, Newington Sittingbourne Kent ME97JL (GB)
(72) Inventor: Lejeune, Béatrice, 44000 Nantes (FR); Floris, Ilaria, 44000 Nantes (FR); Guerra Rodríguez, Mª Carmen, 07330 Consell (ES)
(74) Representative: Clarke Modet & Co.

(57) **Abstract**

The invention refers to compositions and pharmaceutical compositions comprising immunomodulatory agents, and these compositions for use in regenerative medicine treatments, such wound healing and skin repair. In particular, the composition and pharmaceutical composition are for use in micro-immunotherapy.

## Description

### TECHNICAL FIELD

The present invention is related to the medical field. Specifically, to the field of pharmacology and more specifically to pharmaceutical compositions comprising immunomodulatory agents for use in the treatment of diseases.

### BACKGROUND ART

Regeneration and tissue repair processes consist on a sequence of molecular and cellular events which occur after the onset of a tissue lesion in order to restore the damaged tissue. The early exudative phase, then the proliferative and extracellular matrix (ECM) remodelling phases, are sequential events that occur through the integration of dynamic processes involving soluble mediators, blood cells, and parenchymal cells.

During the regeneration and tissue repair processes, the damaged tissue heals by a complex process which involves many cell types and multiple pathways, wherein different cell populations work in a cooperative manner to support a successful tissue regeneration.

After a tissue is damaged (by a traumatic or surgical injury, an infection...) the tissue needs to induce cell proliferation and differentiation to supply tissue-specific cells. The ECM needs to be restored and the vascular, nervous and immune systems must be reestablished, in order to maintain functionality of the new tissue.

Most tissues have fibroblasts in their cellular composition. These cells have mesenchymal origin and are the main producers of ECM components, which are crucial to provide the physical support for other cells to migrate, proliferate, differentiate and perform their specific biological functions.

The immune system is fundamental to determine the quality of the tissue healing response and the restoration of organ structure and function. It is well known that the loss of regenerative capacity is linked to the immunocompetence. The type of immune response, its duration and the cells involved may change the outcome of a tissue healing process from an incomplete healing (scarring or fibrosis) to a complete restoration (healing and tissue repaired).

Micro-immunotherapy (MI) is an immunomodulatory therapy, this is, a therapy which uses compositions comprising the same molecular messengers as the immune system (cytokines, hormones, growth factors, and nucleic acids) along with specific nucleic acids (SNAs®), to modulate the immune response. SNAs® are single stranded DNA molecules from 16 to 34 bases designed to specifically target one or more genes. The main feature of MI is the use of these molecules at low (LD; dilutions of the molecules from 100 times to 10²⁰ times) and ultra-low doses (ULD; dilutions of the molecules higher than 10²⁰ times). These dilutions avoid toxicity and side effects.

It has been suggested the use of compositions of MI in the treatment of rheumatic diseases (Floris I et al. 2018. Journal of Inflammation Research 11;397). However, there is no information in the prior art regarding the use of this medical approach in regenerative medicine and wound healing.

The ability to repair and maintain tissues and organs tends to become less effective in parallel with the age. In addition, inflammation processes during healing usually inhibit the restoration of the tissue towards the promotion of fibrosis.

Regenerative medicine, an interdisciplinary field that applies engineering and life science principles to promote tissue regeneration, is aimed to restore diseased and injured tissues and whole organs. This field holds the premise of healing damaged tissues by stimulating the body's own repair mechanisms. The strategies of regenerative Medicine described are: stem cell therapy, tissue engineering and medical devices/artificial organs.

There is a need of a pharmaceutical composition which improve wound healing, especially in patients having systemic diseases such as endocrine diseases (i.e. diabetes, hypothyroidism), hematologic conditions (i.e. anaemia, polycythemia), cardiovascular problems, gastro-intestinal diseases which cause malnutrition and vitamin deficiencies, obesity or peripheral vascular pathology.

The potential of regenerative medicine in wound healing and tissue repair is enormous. However, there are still ethical, technical and clinical issues that need to be solved before this therapy become routinely used in clinical practice. Despite scientific advances in therapeutic and preventive medicine over the last several decades, regenerative medicine remains expensive and ineffective and has a lot of regulatory drawbacks to be commercialized (Milne CP et al. 2018. Clinical therapeutics, 40; 1056-1059).

Wound-dressing materials and topical agents (foam, hydrogel, hydrofiber or transparent film) are strategies to protect and heal wounds. However, although these approaches may improve wound healing, they have limitations, especially in wounds with high exudates, because wound dressings may lead maceration and bacterial proliferation in the wound (Dhivya S et al. 2015. BioMedicine, 5;4).

The solution provided by traditional wound care is the selection of appropriate dressings in order to maintain a favourable wound healing microenvironment. However, the efficacy of these treatments is even more limited in the so-called "complex wounds", which are difficult to treat (Ferreira MC et al. 2006. Complex wounds. Clinics; 61:571-8). Some wounds heal by primary intention (wound margins brought together and secured), while other wounds are opened and heal from the "bottom up". These wounds are referred to chronic or complex wounds.

Growth factors have been described as a potential treatment in wound healing. However, the effectivity of this approach has been seriously discussed, since the effect of each growth factor may affect differently to the injury due to the completely different growth factor composition of each wound. A "master regulator" of the chronic wound phenotype has not been found yet. Moreover, although scientific reports have pointed the growth factors as a promising strategy, the high production cost of the factors and the difficulties in interpreting the results obtained have delayed experimentation in this field (Han G et al. 2017. Advances in therapy, 34; 599-610).

Therefore, there is a need for an alternative treatment in regenerative medicine, such as wound healing and skin repair able to overcome the problems found in the art. This treatment should not have regulatory issues which could affect to its commercialization and overcome the problems of topic treatments and wound dressings, such as infections, efficiency when exudates are present, and avoid fibrosis. Moreover, this alternative treatment should also be cost-effective.

### DESCRIPTION

The present invention discloses a composition comprising: interleukin 2 (IL-2); epidermal growth factor (EGF); dehydroepiandrosterone (DHEA); dimethyl sulfoxide (DMSO) and RNA molecules. Preferably, the RNA molecules comprise the total RNAfrom *Foeniculum vulgare Miller* ssp *vulgare* and SNA (small nucleic acid) RNAs.

IL-2 (Ref. INTERPRO IPR000779) is a 15.5 - 16 kDa protein which regulates the activities of white blood cells. EGF (Ref. UniProt P01133, UniProt P01132 or UniProtKB P07522) is a growth factor which stimulates cell growth and differentiation by binding to its receptor, EGFR. DHEA (3β-Hydroxyandrost-5-en-17-one) is an endogenous steroid hormone which counteracts the cell immune senescence. DMSO (dimethyl sulphoxyde) repress pro-inflammatory mediators associated with chronic inflammatory disorders.

The RNA molecules present in the composition stimulate the innate immune responses via toll-like receptors activation and generate efficient immune responses.

The composition comprises the total RNA molecules extracted from the leaves of the plant *Foeniculum vulgare* Miller ssp. *vulgare* and the RNA small nucleic acids (SNAs) SNA-HLA I, SNA-HLA II and SNA-TERT.

The total RNA from the leaves of the plant *Foeniculum vulgare* Miller ssp. *vulgare* is obtained protocol described in Claros, M. G., & Canovas, F. M. (1999). RNA isolation from plant tissues: a practical experience for biological undergraduates. Biochemical Education, 27(2), 110-113.

SNA-HLA I refers to an RNA molecule which corresponds to the sequence: SEQ ID NO:1. SNA-HLA-I modulates the expression of HLA-I proteins and avoid the loss of HLA Class-I expression.

SNA-HLA II refers to an RNA molecule or a mixture of RNA molecules selected from any of the following sequences: SEQ ID NO:2, SEQ ID NO:3, SEQ ID NO:4, SEQ ID NO:5, SEQ ID NO:6, or mixtures thereof. SNA-HLA-II molecules counteracts the abnormal levels of expression of HLA-class II molecules related to altered immune functions.

The SNA-TERT refers to an RNA molecule or a mixture of RNA molecules selected from any of the following sequences: SEQ ID NO:7, SEQ ID NO:8 or mixtures thereof. SNA-TERT molecules modulates the expression of human TERT, which plays a balance role between proliferative capacity and tissue regeneration.

The stock concentration of the components of the composition are: 1 µg/mL IL-2, 1 µg/mL EGF, 1 mg/mL DMSO, 1 mg/mL DHEA, 10 µg/mL total RNA *Foeniculum vulgare* Miller ssp. *vulgare*, 80 µg/mL SNA HLA-I, 80 µg/mL SNA HLA-II and 80 µg/mL SNA TERT. Since the International System has not equivalents units for the centesimal Hahnemannian dilutions of the components, the dilutions in this description are expressed in CH from the original stock for each component.

The ingredients of the composition are presented in "centesimal Hahnemannian dilutions" (CH). The centesimal Hahnemannian dilutions CH (or "C scale") represents the number of dilutions of a substance by a factor of 1/100. A 2 CH dilution indicates that a substance has been diluted one hundred times (10⁻²) and then this dilution has been diluted again 1/100 (total dilution: 10⁻⁴). A substance represented by 10 CH indicates that said substance has repeated this process ten times, ending up with the original material diluted by a factor of 10⁻²⁰.

Due to these low/ultra-low doses, the composition of the invention is well tolerated and has no toxicity, which is an advantage, since no toxicity analysis are required for pharmaceutical compositions comprising micro-doses. Article 14 of the Directive 2001/83/EC Of the European Parliament and of the Council of 6 November 2001 on the community code relating to medicinal products for humans, guarantees the safety of medical products that contain "no more than one part per 10,000 (2 CH) of the mother tinctures, or no more than a hundredth part of the smallest dose used in allopathy with regard to active substances whose presence in an allopathic medicinal product results in the obligation to submit a doctor's prescription".

In an embodiment, the composition comprises the following components: DMSO, SNA-HLAI, SNA-HLAII, RNA, DHEA, IL-2, EGF and SNA-TERT in dilutions ranging from 2 to 30 CH.

In a preferred embodiment, the composition comprises 8-12 CH of IL-2 (1 µg/mL IL-2 diluted 10⁻¹⁶ - 10⁻²⁴ times); 8-12 CH of EGF(1 µg/mL EGF diluted 10⁻¹⁶ - 10⁻²⁴ times); 8-10 CH of DHEA (1 mg/mL DHEA diluted 10⁻¹⁶ - 10⁻²⁰ times); 8-10 CH of DMSO (1 mg/mL DMSO diluted 10⁻¹⁶ - 10⁻²⁰ times); 8-10 CH of total RNA *F. vulgare* (10 µg/mL total RNA diluted 10⁻¹⁶ - 10⁻²⁰ times); 8-10 CH of SNA-HLA-I (80 µg/mL SNA-HLA-I diluted 10⁻¹⁶ -10⁻²⁰ times); 8-10 CH of SNA-HLA-II (80 µg/mL SNA-HLA-II diluted 10⁻¹⁶ - 10⁻²⁰ times) and 8-16 CH of SNA-TERT (80 µg/mL SNA-TERT diluted 10⁻¹⁶ - 10⁻³² times).

In a more preferred embodiment, the pharmaceutical composition comprises 10 CH of IL-2 (1 µg/mL IL-2 diluted 10⁻²⁰ times), 10 CH of EGF (1 µg/mL EGF diluted 10⁻²⁰ times), 10 CH of DHEA (1 mg/mL IL-2 diluted 10⁻¹⁶ - 10⁻²⁴ times), 10 CH of DMSO (1 mg/mL DMSO diluted 10⁻²⁰ times), 10 CH of total *F. vulgare* RNA (10 µg/mL total RNA diluted 10⁻²⁰ times), 10 CH of SNA-HLA-I (80 µg/mL SNA-HLA-I diluted 10⁻²⁰ times), 10 CH of SNA-HLA-II (80 µg/mL SNA-HLA-II diluted 10⁻²⁰ times) and 16 CH of SNA-TERT (80 µg/mL SNA-TERT diluted 10⁻³² times).

In a preferred embodiment, SNA-HLA I is a sequence comprising SEQ ID NO:1. In a more preferred embodiment, the sequence of SNA-HLA I is SEQ ID NO:1.

In a preferred embodiment, SNA-HLA II comprises any of the following sequences SEQ ID NO:2, SEQ ID NO:3, SEQ ID NO:4, SEQ ID NO:5, or SEQ ID NO:6, or a mixture thereof. In a more preferred embodiment, SNA-HLA II comprises a sequence consisting of any of the following sequences: SEQ ID NO:2, SEQ ID NO:3, SEQ ID NO:4, SEQ ID NO:5, or SEQ ID NO:6, or a mixture thereof. In an even more preferred embodiment, SNA-HLA II is the mixture of SEQ ID NO:2, SEQ ID NO:3, SEQ ID NO:4, SEQ ID NO:5 and SEQ ID NO:6.

In a preferred embodiment, SNA-TERT comprises any of the following sequences: SEQ ID NO:7 or SEQ ID NO:8, or a mixture thereof. In a more preferred embodiment, SNA-TERT comprises a sequence consisting on any of the following sequences: SEQ ID NO:7 or SEQ ID NO:8, or a mixture thereof. In an even more preferred embodiment, SNA-TERT is the mixture of SEQ ID NO:7 and SEQ ID NO:8.

In a preferred embodiment, the composition disclosed in any of the previous embodiments further comprises an auxiliary agent, which may be selected from the group comprising emulsifiers, salts, pharmacologically acceptable buffers, binders, glidants, lubricants, disintegrants, sweeteners, pigments, co-solvents, surfactants, oils, humectants, emollients, preservatives, stabilizers, antioxidants or combinations thereof.

It has been shown that the composition described herein plays a regulatory role in different physio-pathological pathways related to cellular regeneration and wound healing, promoting cellular regeneration.

In an embodiment, the invention discloses a pharmaceutical composition comprising the composition defined in any of the previous embodiments and a pharmaceutically-acceptable carrier. Pharmaceutically acceptable carriers are non-toxic excipients which may include vehicles commonly used to form pharmaceutical compositions for animal or human administration. The pharmaceutically-acceptable carrier is selected so as not to unduly affect the biological activity of the composition of the invention.

In a preferred embodiment, the composition or pharmaceutical composition disclosed in any of the previous embodiments is in combination with another regenerative medicine treatment. Another regenerative medicine treatment is defined as any treatment of regenerative medicine, including wound dressings (such as gauze, films, hydrocolloids, hydrogels, foams, alginates or hydrofibers), tissue engineered skin substitutes, negative pressure wound therapy, growth factors or hyperbaric oxygen, among others. The composition of the invention supports the physiological processes of tissue repair and regeneration after an injury, improving the tissue homeostasis and its repair. Wound healing is a complex process, which involves many cell types and mediators interacting in a temporal sequence, aimed at restoring the structural and functional integrity of the skin as a barrier to external stressors. Progression from an acute to chronic wound would be the result of local factors (such as tissue ischemia, infection, the presence of necrotic tissue or ionizing radiation) or systemic factors (such as diabetes, smoking or malnutrition). These factors alter the wound microenvironment.

In another embodiment, the invention is directed to a composition or a pharmaceutical composition as defined above for use as medicament.

In another embodiment, the invention is directed to a composition or a pharmaceutical composition as defined above, for use in the treatment of a disease in need of wound healing and/or skin repair.

An aim of the composition or pharmaceutical composition of the present invention to help to prevent chronic wound in aged persons and in patients having systemic diseases such as endocrine diseases (such as diabetes or hypothyroidism), hematologic conditions (such as anaemia or polycythemia), cardiovascular diseases, gastro-intestinal diseases, obesity or peripheral vascular pathology. In the present invention, the expressions wound healing and skin repair may be considered synonyms.

In another embodiment, composition or the pharmaceutical composition as defined above, is for use in Micro-immunotherapy (MI).

The composition of the invention helps in the restoration, maintenance or improving tissue function and tissue repair in adult/elderly population, and also sustaining wound healing and regeneration after injury in young population.

In an embodiment, the invention is also directed to the use of the composition or the pharmaceutical composition as described above, in manufacturing a medicament for the treatment of regenerative medicine, preferably wound healing and/or skin repair.

In an embodiment, the invention is directed to a method of treatment which comprises the administration of an effective amount of the pharmaceutical composition described above.

In an embodiment, the invention is directed to the composition or the pharmaceutical composition described above, for use in a method of treatment of a disease selected from wound healing and/or skin repair, wherein the composition is administered in a single dose (or single administration) or as part of a sequential administration For purposes of the present invention, a sequential administration is defined as the administration of a pharmaceutical composition having the same ingredients and active principles, but in different concentrations from each other.

The physical form of the pharmaceutical composition may be solid, semisolid, liquid or gaseous. For preparing solid compositions, the compound of interest is mixed or impregnated into formulations with conventional ingredients such as talc, magnesium stearate, dicalcium phosphate, magnesium aluminium silicate, calcium sulphate, starch, sucrose, lactose, acacia, methylcellulose, kaolin and functionally similar materials as pharmaceutical diluents or carriers. In a preferred embodiment, the pharmaceutical composition is in solid form. In a more preferred embodiment, the pharmaceutical composition is impregnated in or mixed with lactose-sucrose globules.

An embodiment of the invention refers to the route of administration of the pharmaceutical composition defined above. The pharmaceutical composition of the invention may be administered to a subject in need thereof via oral, nasal, topical, pulmonary, transdermal or parenteral, rectal, subcutaneous, intravenous, intraurethral, intramuscular, intranasal or ophthalmic administration. For topical administration, the composition may be mixed with a pharmaceutically acceptable vehicle such as water, alcohol, oils, fats and mixtures thereof. The pharmaceutical composition in liquid form for injectable formulations may be diluted in a pharmaceutically acceptable diluent. Examples of such diluents are water, saline, organic or inorganic salt solutions, Ringer's solution, dextrose solution, and Hank's solution. In a preferred embodiment, the pharmaceutical composition is administered sublingually.

In a preferred embodiment, the compositions of the invention are administered orally. This kind of administration avoids all the inconvenient of topical treatments, Oral administration is considered safer, cheaper, painless, convenient for repeated and prolonged use and can be self-administered. For these reasons, patients prefer oral administration.

In an embodiment, the pharmaceutical composition may be presented in any dosage form known in the art, such as tablets, capsules, lozenge, pastilles, pills, granules, powder, cream, ointment, gel, poultice, paste, liniment, wafers, suppositories, cachets, teaspoonfuls, tablespoonfuls, dropperfuls, ampoules, vials, aerosols with metered discharges, segregated multiples of any of the foregoing, and other forms as herein described. In a preferred embodiment, the pharmaceutical composition of the invention is presented in form of tablets or capsules. Capsules are prepared by mixing the ingredients with an inert pharmaceutical diluent and filling the mixture into a hard gelatine capsule of appropriate size. Soft gelatine capsules are prepared by machine encapsulation of slurry of the compound of interest with an acceptable vegetable oil, light liquid petrolatum or other inert oil.

In an embodiment, the invention refers to a kit of parts comprising one or more unit-dosage forms of the composition defined in the previous embodiments, and instructions for use to treat one or more of the disorders described herein.

In an embodiment, the pharmaceutical composition is administered in the morning at least 15 min before the ingestion of any food. The morning is considered between 1:00. and 14:00 (24-hour format time).

Throughout the description and the claims, the term "comprises" and its variants does not exclude other technical characteristics, additives, components or steps. For experts in the field, other purposes, advantages and characteristics of the invention will derive in part from the description and in part from the practice of the invention.

### BRIEF DESCRIPTION OF DRAWINGS

**Figure 1****.** Percentage of cell death after 12 hours of treatment in two MTT assays. It is represented mean and standard deviation of 6 to 7 replicates. Grey bars correspond to placebo and white bars correspond to the pharmaceutical composition (** p < 0,01).
**Figure 2****.** Percentage of cell death after 12 hours of treatment in two MTT assays. It is represented mean and standard deviation of 5 to 7 replicates. Grey bars correspond to placebo and white bars correspond to the pharmaceutical composition (** p < 0,01).
**Figure 3****.** Percentage of cell death after 12 hours of treatment in two MTT assays. It is represented mean and standard deviation of 5 to 7 replicates. Grey bars correspond to placebo and white bars correspond to the pharmaceutical composition (* p < 0,5; ** p < 0,01; *** p < 0,001).
**Figure 4****.** Wound healing capacity of the composition of the invention. Measurement of the relative wound density (%) over time. The samples analysed were the negative control (-○-, white), a composition comprising EGF 250 ng/mL (-●-, black), placebo ( 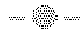 , soft grey) and the composition of the invention ( 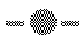 , dark grey)
**Figure 5****.** Images of wound healing capacity of the composition of the invention in over time (at 0 h, 24 h and 48 hours) compared to the placebo. The images were taken using a live content cell imaging system.

### DESCRIPTION OF THE SEQUENCE LISTING

The present invention discloses the following sequences:
- SEQ ID NO:1: SNA-HLA II-1 (caauaauccg gccccucc)
- SEQ ID NO:2: SNA-HLA II-2 (ccguugguga agua)
- SEQ ID NO:3: SNA-HLA II-3 (ggcccgccug ucuucca)
- SEQ ID NO:4: SNA-HLA II-4 (cucccccacg ucgcuguc)
- SEQ ID NO:5: SNA-HLA II-5 (guugacacau gguccg)
- SEQ ID NO:6: SNA-HLA II-6 (augagcggag gcgcca)
- SEQ ID NO:7: SNA-TERT - 1 (caggaacttg gccaggatct cctc)
- SEQ ID NO:8: SNA-TERT - 2 (cggccggaac acagccaac)

### DESCRIPTION OF EMBODIMENTS

The following examples and figures are provided for illustration purposes and are not intended to be limiting of the present invention.

### Example 1: Preparation of the composition

The composition is prepared by mixing the specified amounts of the following ingredients:
- IL-2: 10 CH;
- EGF: 10 CH;
- DHEA: 10 CH;
- DMSO: 10 CH;
- RNA: 10 CH;
- SNA-HLA-I: 10 CH;
- SNA-HLA-II: 10 CH; and
- SNA-TERT: 16 CH.

The concentration of the ingredients of the composition are expressed as CH (Centesimal Hahnemannian dilutions). The stock concentration of the components of the composition are: 1 µg/mL IL-2, 1 µg/mL EGF, 1 mg/mL DMSO, 1 mg/mL DHEA, 10 µg/mL total RNA *Foeniculum vulgare* Miller ssp. *vulgare*, 80 µg/mL SNA HLA-I, 80 µg/mL SNA HLA-II and 80 µg/mL SNA TERT. Since the International System has not equivalents units for the centesimal Hahnemannian dilutions used in the invention, the dilutions are expressed in CH from the original stock for each component.

For this experiment, IL-2 was produced under GMP conditions in LABO'LIFE ESPAÑA S.A. laboratory by recombinant DNA technology using *Escherichia coli* as host. EGF was provided by Peprotech (Catalog n. AF-100-15). DHEA was provided by Bayer and DMSO was provided by Gaylord Chemical Company LLC.

The RNA corresponds to total RNA extracted from the leaves of the plant *Foeniculum vulgare Miller* ssp *vulgare.* RNA has been extracted through isolation, purification and elution according to Claros, MG & Canovas FM (1999). RNA isolation from plant tissues: a practical experience for biological undergraduates. Biochemical Education, 27(2), 110-113).

The RNA extracted was quantified and its purity was measured with a Nanodrop by absorbance and checking the ratio A260/A230 and A260/280.

All SNAs were manufactured by EUROGENTEC S.A.

The sequence of SNA-HLA-I corresponds to the sequence defined in SEQ ID NO:1. The sequences of SNA-HLA-II corresponds to the sequence defined in SEQ ID NO:2, SEQ ID NO:3, SEQ ID NO:4, SEQ ID NO:5 and SEQ ID NO:6. The sequence of SNA-TERT corresponds to the sequence defined in SEQ ID NO:7 and SEQ ID NO:8.

### Example 2: Effect of the composition on fibroblast viability

In order to investigate the *in vitro* efficacy of the composition, it was performed an experiment on human dermal adult fibroblasts (HDAF), comparing the composition with a placebo. HDAF represent an excellent model system to study cell physiology related to skin biology, wound healing and skin aging. Fibroblasts are an immune competent cell type which plays a critical role in chronic inflammation. These cells act as "*sentinel cells*" in the immune system and are involved in the switch from acute inflammation to adaptive immunity and tissue repair.

The survival response of human primary dermal fibroblasts was analysed by treating the cells with lactose-sucrose globules impregnated with the composition of the invention described in example 1. The impregnation process occurs as follows:
1. The impregnation flask with the specific weight of globules needed is placed on a rotational module;
2. The composition is dispensed through a pipette in the impregnation flask over the globules while the flask rotates;
3. After the impregnation process, a further dry step is conducted (also while the flask rotates).
The impregnation concentration of the composition used in each globule is 1 % (w/w).

The placebo capsules contained lactose-sucrose globules impregnated without the composition. 0,38 g of lactose and sucrose was used as excipient in both the composition and the placebo. The composition and the placebo were both kept at room temperature, away from light and moisture until their use.

Primary cultures of human dermal adult fibroblasts, HDAF (ATCC PCS-201-012) were seeded at 3 x 10³ cells/cm² in GlutaMaxTM high glucose Dulbecco's modified Eagle's medium (DMEM, Gibco®), and supplemented with 10 % fetal bovine serum (FBS, HyClone, Thermo Scientific), penicillin (Pen) 100 U/mL and streptomycin (Strep) 1000 U/mL.

Cells were passaged at sub-confluence (70 - 80 %) every 4-5 days. All incubation steps were carried out in a temperature and humidity controlled in an incubator at 37 °C in 5 % CO₂.

The globules were diluted in pre-warmed DMEM (in absence of FBS and Pen/Strep), and seven point-curves (serial three-fold dilutions) were prepared at different lactose-sucrose concentrations ranging from 3,8 mg/mL to 0,005 mg/mL (760 times less concentrated). HDAF were treated for 12, 24 and 48 hours.

The cells were seeded in 96 well plates (Nunc) and treated with the composition or with the placebo. 1,5 × 10⁴ cells/well were incubated for 12, 24 or 48 hours. Previous experiments have shown that this cell concentration is the best for MTT assays, with better sensitivity and low variability. Samples were run per triplicates or quadruplicates.

After incubation, the cell media was replaced with MTT reagent consisting of thiazole blue tetrazolium bromide (Sigma # 298-93-1) at 0,5 mg/mL in DMEM without phenol red. The plates were gently shaken and incubated for 3 hours at room temperature. The, the medium was removed and replaced by 100 µL of DMSO. The plates were gently shaken to solubilize the formazan crystals. Absorbance was measured using an Envision multiplate reader at a wavelength of 570 nm.

Cell viability tests are fundamental for evaluation of cell health, compound toxicity and drug efficacy. Cell viability can be detected based on many different parameters: the redox potential of the cell population, the integrity of cell membranes, or the activity of cellular enzymes. MTT assay is a common method applied for evaluation of cell viability and cytotoxicity for drug screening.

The reagent used is thiazole blue tetrazolium bromide (Sigma # 298-93-1), a positively charged small molecule that can enter in viable cells and non-viable cells and that undergoes NADPH-mediated conversion over to formazan. Upon conversion, the formazan product precipitates inside cells, near the cell surface, and can be detected using a spectrophotometer. MTT only needs an intact and functioning mitochondrion to be converted, defining it as a metabolic assay and not as a proliferation assay.

Statistical analysis was performed using GraphPad Prism version 7.0. The % of cell death induced by the placebo globules was compared to the corresponding concentration of the capsule globules of the invention using unpaired Mann-Whitney non-parametric tests (p < 0,01 **).

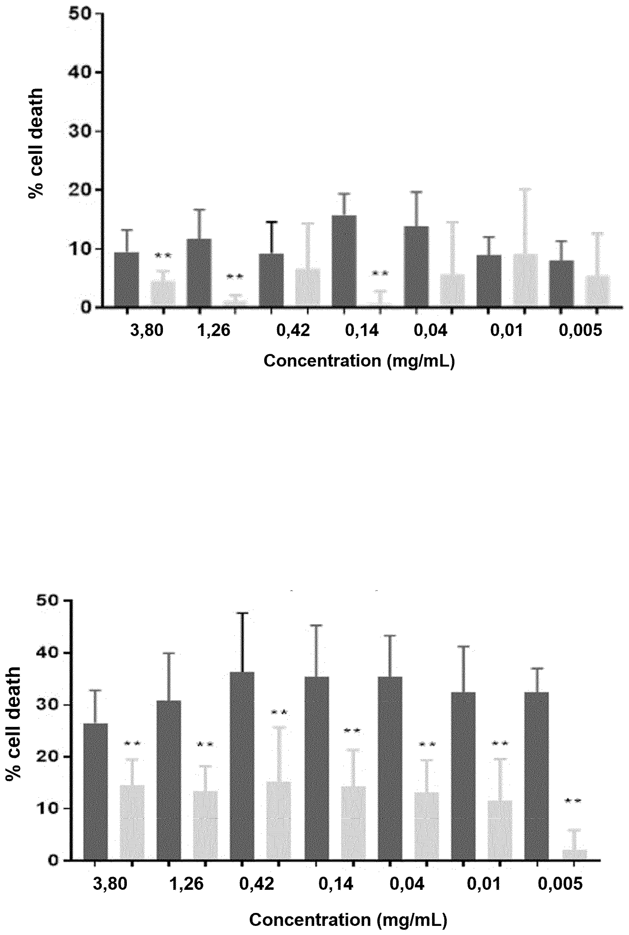

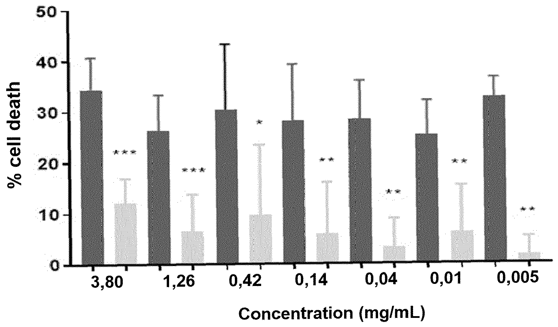

During the assay, the cells were observed under an optical microscope and, in all cases, they presented normal appearance. At 12 hours, both treatments, placebo and the composition of the invention showed a high percentage of cell survival (maximum value of cell death 25%) (Figure 1). Cell survival decreased after 24 and 48 hours of treatment (maximum value of cell death 54%) (Figures 2 and 3).

Tables 1, 2 and 3 show respectively the results of each MTT experiment (12, 24 or 48 hours) showing that he composition of the invention showed less toxicity (lower % of cell death) than placebo at all-time points.

**Table 1: MTT assay at 12 hours**

| **MTT (12 hours)** | **N values** | **Min** | **Max** | **Mean** | **Std. Dev** | **Std. Err** |
|---|---|---|---|---|---|---|
| Placebo (3,80 mg/mL) | 7 | 5,91 | 16,59 | 9,57 | 3,66 | 1,38 |
| Composition (3,80 mg/mL) | 6 | 1,97 | 7,076 | 4,53 | 1,77 | 0,72 |
| Placebo (1,26 mg/mL) | 6 | 7,01 | 20,07 | 11,72 | 4,95 | 2,02 |
| Composition (1,26 mg/mL) | 6 | 0 | 2,42 | 1,10 | 1,02 | 0,41 |
| Placebo (0,42 mg/mL) | 7 | 2,64 | 16,86 | 9,32 | 5,25 | 1,98 |
| Composition (0,42 mg/mL) | 7 | 0,35 | 18 | 6,66 | 7,63 | 2,88 |
| Placebo (0,14 mg/mL) | 7 | 9,88 | 20,88 | 15,77 | 3,59 | 1,36 |
| Composition (0,14 mg/mL) | 6 | 0 | 4,81 | 0,80 | 1,96 | 0,80 |
| Placebo (0,04 mg/mL) | 6 | 5,32 | 23,29 | 13,81 | 5,87 | 2,39 |
| Composition (0,04 mg/mL) | 7 | 0 | 21,36 | 5,70 | 8,84 | 3,34 |
| Placebo (0,01 mg/mL) | 7 | 5,64 | 14,98 | 8,94 | 3,12 | 1,18 |
| Composition (0,01 mg/mL) | 7 | 1,38 | 25,23 | 9,23 | 10,97 | 4,14 |
| Placebo (0,005 mg/mL) | 6 | 4,82 | 13,91 | 8,08 | 3,30 | 1,35 |
| Composition (0,005 mg/mL) | 7 | 0 | 17,74 | 5,56 | 7,03 | 2,65 |

At 12 hours of treatment the composition significantly reduced the percentage of cell death compared to the placebo at all concentrations, from 3,8 mg/mL to 0,005 mg/mL (p<0,01).

**Table 2: MTT assay at 24 hours**

| **MTT (24 hours)** | **N values** | **Min** | **Max** | **Mean** | **Std. Dev** | **Std. Err** |
|---|---|---|---|---|---|---|
| Placebo (3,80 mg/mL) | 7 | 19,72 | 36,76 | 26,51 | 6,25 | 2,36 |
| Composition (3,80 mg/mL) | 7 | 6,74 | 20,41 | 14,49 | 4,96 | 1,87 |
| Placebo (1,26 mg/mL) | 7 | 16,57 | 39,95 | 30,77 | 9,16 | 3,46 |
| Composition (1,26 mg/mL) | 7 | 3,14 | 17 | 13,37 | 4,85 | 1,83 |
| Placebo (0,42 mg/mL) | 7 | 22,47 | 54,44 | 36,33 | 11,35 | 4,29 |
| Composition (0,42 mg/mL) | 7 | 5,06 | 37,19 | 15,16 | 10,49 | 3,96 |
| Placebo (0,14 mg/mL) | 7 | 20,47 | 47,48 | 35,26 | 10,03 | 3,79 |
| Composition (0,14 mg/mL) | 7 | 3,86 | 27,12 | 14,16 | 7,13 | 2,69 |
| Placebo (0,04 mg/mL) | 6 | 25,77 | 46,76 | 35,29 | 8,01 | 3,27 |
| Composition (0,04 mg/mL) | 6 | 1,46 | 19,3 | 13,02 | 6,26 | 2,55 |
| Placebo (0,01 mg/mL) | 7 | 14,78 | 40,14 | 32,35 | 8,88 | 3,35 |
| Composition (0,01 mg/mL) | 7 | 0 | 25,2 | 11,38 | 8,18 | 3,09 |
| Placebo (0,005 mg/mL) | 5 | 27,29 | 39,01 | 32,37 | 4,61 | 2,06 |
| Composition (0,005 mg/mL) | 6 | 0 | 9,80 | 1,976 | 3,92 | 1,6 |

**Table 3: MTT assay at 48 hours**

| **MTT (48 hours)** | **N values** | **Min** | **Max** | **Mean** | **Std. Dev** | **Std. Err** |
|---|---|---|---|---|---|---|
| Placebo (3,80 mg/mL) | 7 | 24,43 | 41,91 | 34,51 | 6,25 | 2,36 |
| Composition (3,80 mg/mL) | 7 | 6,74 | 20,41 | 12,24 | 4,60 | 1,74 |
| Placebo (1,26 mg/mL) | 7 | 16,57 | 37,47 | 26,21 | 7,14 | 2,7 |
| Composition (1,26 mg/mL) | 7 | 0 | 16,33 | 6,43 | 7,27 | 2,74 |
| Placebo (0,42 mg/mL) | 7 | 18,7 | 54,44 | 30,39 | 12,89 | 4,87 |
| Composition (0,42 mg/mL) | 7 | 0 | 37,19 | 9,68 | 13,74 | 5,19 |
| Placebo (0,14 mg/mL) | 7 | 13,84 | 47,48 | 28,13 | 11,11 | 4,2 |
| Composition (0,14 mg/mL) | 7 | 0 | 27,12 | 5,90 | 10,11 | 3,82 |
| Placebo (0,04 mg/mL) | 6 | 21,4 | 37,96 | 28,35 | 7,58 | 3,09 |
| Composition (0,04 mg/mL) | 6 | 0 | 14,65 | 3,03 | 5,76 | 2,35 |
| Placebo (0,01 mg/mL) | 7 | 14,78 | 36,4 | 25,07 | 6,89 | 2,60 |
| Composition (0,01 mg/mL) | 7 | 0 | 25,2 | 6,09 | 9,26 | 3,5 |
| Placebo (0,005 mg/mL) | 5 | 27,29 | 37,84 | 32,63 | 3,89 | 1,74 |
| Composition (0,005 mg/mL) | 5 | 0 | 8,08 | 1,62 | 3,61 | 1,61 |

At 48 hours of treatment, the composition significantly reduced the % of cell death compared to placebo at all concentrations (p<0,001 at 3,8 and 1,26 mg/mL; p<0,01 from 0,14 to 0,005 mg/mL; p<0,05 at 0,42 mg/mL).

All concentrations of the composition reduce the percentage of cells death, which make the composition suitable for use in tissue regeneration and wound healing.

### Example 3: Effect of the composition in wound healing

A primary culture of HDAF as defined in Example 2 (N=3) was cultured in DMEM medium containing 10% FBS and 1% Penicillin-Streptomycin (Sigma). Cells were used at passage 3 for the real time wound-healing assay, a technique used to study cell migration, wound healing *in vitro* and cell-cell interaction. This assay is characterized by the scratch on a cell monolayer which is made to produce a cell-free area in hopes of inducing cells to migrate and close the gap. Capturing images at regular intervals by time lapse microscope is it possible to quantify the speed and the capacity to close and repair the scratch.

Cells were cultured in a 96-well Essen ImageLock plate (Essen BioScience) and grown to confluence in a CO₂ humidified incubator.

Then, every well was scratched using a 96-pin WoundMaker (Essen BioScience), which makes fast and uniform wounds across various numbered wells.

The growth medium was removed and replaced with:
- DMEM without FBS (negative control);
- placebo globules dissolved in DMEM without FBS at 7.6 mg/mL (placebo control);
- active globules impregnated with the composition of the invention described in example 1, were dissolved in DMEM without FBS at 7.6 mg/mL;
- rhEGF (Immunotools catalogue number 11343406) in DMEM without FBS at 250 ng/mL was used as positive control to induce a clear and significant wound healing activity.

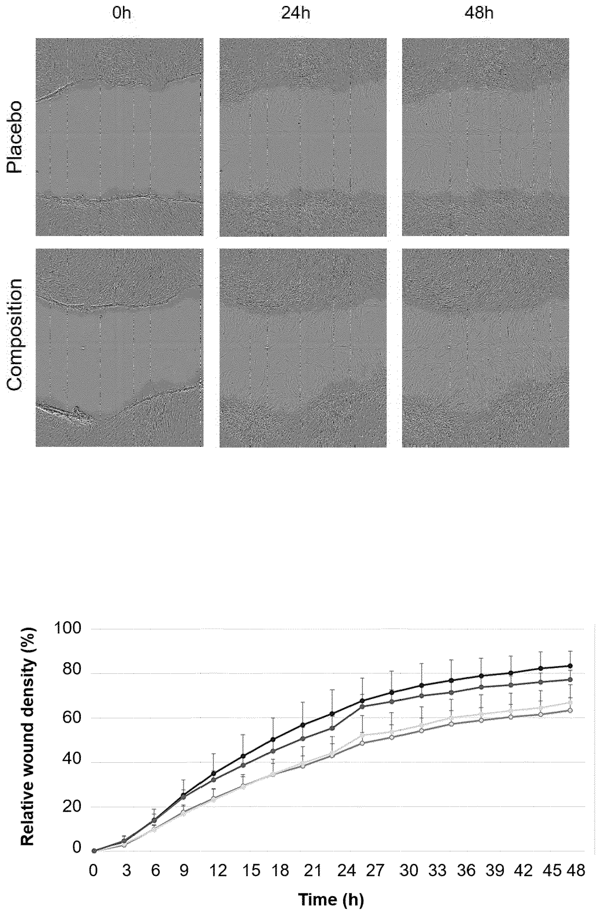

Once the medium was replaced and each treatment started, images were taken using the live content cell imaging system IncuCyte HD (Essen BioScience) (time zero). Images were taken every 3 h for 24 h, then medium was removed, and fresh media was added, and each sample was incubated for another 24 h, with same culture conditions. Figure 4 shows the images at 0, 24 and 48 hours of the composition and the placebo samples.

This experiment was run in triplicate. Data were analysed by the integrated metric Relative Wound Density, part of the live content cell imaging system IncuCyte HD (Essen BioScience).

Statistical analysis was performed using Statistical Analysis Software (SAS). The relative wound healing density measured in placebo treated cells was compared to correspondent concentration of composition treated cells, using unpaired t student tests. Mean, standard deviation and P values of both groups are listed in **Table 4.**

**Table 4: Relative wound healing density (mean and standard deviation) measured in placebo and composition treated cells. P values were obtained using SAS, and unpaired t Student test was performed to analyse statistical differences between the two groups.**

| **Time (hours)** | **Placebo Mean (Stdev)** | **Composition Mean (Stdev)** | **P value** |
|---|---|---|---|
| Time 3 | 3.247 (1.365) | 4.639 (1.885) | 0.0917 |
| Time 6 | 9.562 (1.953) | 13.794 (2.913) | 0.0023 |
| Time 9 | 16.704 (3.389) | 24.172 (3.212) | 0.0002 |
| Time 12 | 23.157 (4.944) | 32.086 (3.919) | 0.0006 |
| Time 15 | 28.961 (5.381) | 38.665 (3.946) | 0.0005 |
| Time 18 | 34.606 (6.710) | 44.917 (4.467) | 0.0015 |
| Time 22 | 39.612 (7.345) | 50.530 (4.993) | 0.0020 |
| Time 24 | 43.992 (7.359) | 55.219 (5.263) | 0.0019 |
| Time 27 | 51.958 (8.948) | 65.001 (5.380) | 0.0018 |
| Time 30 | 53.597 (8.657) | 67.183 (5.254) | 0.0010 |
| Time 33 | 56.501 (8.362) | 69.835 (5.303) | 0.0009 |
| Time 36 | 59.846 (8.331) | 71.227 (4.723) | 0.0026 |
| Time 39 | 61.639 (8.614) | 73.817 (5.235) | 0.0023 |
| Time 42 | 63.066 (7.986) | 74.658 (4.092) | 0.0013 |
| Time 45 | 64.312 (7.743) | 75.919 (4.133) | 0.0011 |
| Time 48 | 66.719 (8.118) | 77.146 (4.153) | 0.0034 |

As shown in Table 4 and also in Figure 5, the composition (-●-) increases the relative wound healing capacity *in vitro* compared to the placebo ( 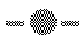 ), from 6 hours of treatment, in all time-points during treatment (until 48 hours).

These results show that the composition is suitable for use in tissue repair and wound healing.

## Claims

1. Composition comprising 8-12 CH of IL-2 (1 µg/mL of IL-2 diluted 10⁻¹⁶ - 10⁻²⁴ times); 8-12 CH of EGF (1 µg/mL of EGF diluted 10⁻¹⁶ - 10⁻²⁴ times); 8-10 CH of DHEA (1 mg/mL of DHEA diluted 10⁻¹⁶- 10⁻²⁰ times); 8-10 CH of DMSO (1 mg/mL of DMSO diluted 10⁻¹⁶ - 10⁻²⁰ times); 8-10 CH of total RNA *F. vulgare* (10 µg/mL total RNA diluted 10⁻¹⁶ - 10⁻²⁰ times); 8-10 CH of SNA-HLA-I (80 µg/mL of SNA-HLA-I diluted 10⁻¹⁶ - 10⁻²⁰ times); 8-10 CH of SNA-HLA-II (80 µg/mL SNA-HLA-II diluted 10⁻¹⁶ - 10⁻²⁰ times) and 8-16 CH of SNA-TERT (80 µg/mL of SNA-TERT diluted 10⁻¹⁶ - 10⁻³² times).

2. The composition of claim 1, comprising CH of IL-2 (1 µg/mL of IL-2 diluted 10⁻²⁰ times), 10 CH of EGF (1 µg/mL of EGF diluted 10⁻²⁰ times), 10 CH of DHEA (1 mg/mL of DHEA diluted 10⁻¹⁶ - 10⁻²⁴ times), 10 CH of DMSO (1 mg/mL of DMSO diluted 10⁻²⁰ times), 10 CH of total RNA *F. vulgare* (10 µg/mL of total RNA diluted 10⁻²⁰ times), 10 CH of SNA-HLA-I (80 µg/mL of SNA-HLA-I diluted 10⁻²⁰ times), 10 CH of SNA-HLA-II (80 µg/mL of SNA-HLA-II diluted 10⁻²⁰ times) and 16 CH of SNA-TERT (80 µg/mL of SNA-TERT diluted 10⁻³² times).

3. The composition of claims 1 or 2, wherein the sequence of SNA-HLA I comprises SEQ ID NO:1.

4. The composition of any of claims 1 to 3, wherein the sequence of SNA-HLA I is SEQ ID NO:1.

5. The composition of any of claims 1 to 4, wherein SNA-HLA II comprises the following sequences SEQ ID NO:2, SEQ ID NO:3, SEQ ID NO:4, SEQ ID NO:5, or SEQ ID NO:6, or a mixture thereof.

6. The composition of claim 5, wherein SNA-HLA II comprises the mixture of SEQ ID NO:2, SEQ ID NO:3, SEQ ID NO:4, SEQ ID NO:5 and SEQ ID NO:6.

7. The composition of any of claims 1 to 6, wherein SNA-TERT comprises SEQ ID NO:7 or SEQ ID NO:8, or a mixture thereof.

8. The composition of claim 7, wherein SNA-TERT comprises the mixture of SEQ ID NO:7 and SEQ ID NO:8.

9. The composition of any of claims 1 to 8 further comprising an auxiliary agent selected from an emulsifier, salt, buffer, colouring substance or combinations thereof.

10. Pharmaceutical composition comprising the composition of any one of claims 1 to 9 and a pharmaceutically acceptable carrier.

11. The composition or pharmaceutical composition of any of claims 1 to 10 for use as medicament.

12. The composition or pharmaceutical composition according to any one of claims 1 to 10 for use in the treatment of a disease selected from wound healing and/or skin repair.

13. The composition or pharmaceutical composition of any one of claims 1 to 10 for use in micro-immunotherapy.

14. The composition or the pharmaceutical composition of any of claims 1 to 10 for use in the treatment of a disease selected from wound healing and/or skin repair, wherein the composition is administered in a single dose or as part of a sequential administration.

15. The composition or pharmaceutical composition according to any one of claims 1 to 10, in combination with a regenerative medicine treatment.


SEQUENCE LISTING

<110> LABO'LIFE

<120> MICRO-IMMUNOTHERAPY COMPOSITIONS AND THEIR USES IN WOUND HEALING AND
TISSUE REPAIR

<130> 2017/92341

<160> 8

<170> BiSSAP 1.3.6

<210> 1
<211> 18
<212> RNA
<213> Artificial Sequence

<220>
<223> SNA-HLA II-1

<400> 1
caauaauccg gccccucc                                                    18

<210> 2
<211> 14
<212> RNA
<213> Artificial Sequence

<220>
<223> SNA-HLA II-2

<400> 2
ccguugguga agua                                                        14

<210> 3
<211> 17
<212> RNA
<213> Artificial Sequence

<220>
<223> SNA-HLA II-3

<400> 3
ggcccgccug ucuucca                                                     17

<210> 4
<211> 18
<212> RNA
<213> Artificial Sequence

<220>
<223> SNA-HLA II-4

<400> 4
cucccccacg ucgcuguc                                                    18

<210> 5
<211> 16
<212> RNA
<213> Artificial Sequence

<220>
<223> SNA-HLA II-5

<400> 5
guugacacau gguccg                                                    16

<210> 6
<211> 16
<212> RNA
<213> Artificial Sequence

<220>
<223> SNA-HLA II-6

<400> 6
augagcggag gcgcca                                                    16

<210> 7
<211> 24
<212> DNA
<213> Artificial Sequence

<220>
<223> SNA-TERT - 1

<400> 7
caggaacttg gccaggatct cctc                                           24

<210> 8
<211> 19
<212> DNA
<213> Artificial Sequence

<220>
<223> SNA-TERT - 2

<400> 8
cggccggaac acagccaac                                                 19